(19) Europäisches Patentamt / European Patent Office / Office européen des brevets

(11) **EP 3 749 278 B1**

(12) **EUROPEAN PATENT SPECIFICATION**

(45) Date of publication and mention
of the grant of the patent:
**13.10.2021  Bulletin 2021/41**

(21) Application number: **19701245.3**

(22) Date of filing: **28.01.2019**

(51) Int Cl.:
*A61K 8/25* (2006.01)          *A61K 8/26* (2006.01)
*A61K 8/28* (2006.01)          *A61K 8/29* (2006.01)
*A61Q 11/00* (2006.01)          *A61K 8/19* (2006.01)

(86) International application number:
**PCT/EP2019/051943**

(87) International publication number:
**WO 2019/154646 (15.08.2019 Gazette 2019/33)**

(54) **ORAL CARE COMPOSITION**

MUNDPFLEGEZUSAMMENSETZUNG

COMPOSITION D'HYGIÈNE BUCCALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **09.02.2018  PCT/CN2018/075937
12.04.2018  EP 18166970**

(43) Date of publication of application:
**16.12.2020  Bulletin 2020/51**

(73) Proprietors:
• **Unilever Global IP Limited
Wirral, CH62 AZD (GB)**
Designated Contracting States:
**CY DE GB IE IT MT**
• **Unilever IP Holdings B.V.
3013 AL Rotterdam (NL)**
Designated Contracting States:
**AL AT BE BG CH CZ DK EE ES FI FR GR HR HU
IS LI LT LU LV MC MK NL NO PL PT RO RS SE SI
SK SM TR**

(72) Inventors:
• **DONG, Wenyan
Shanghai, 200335 (CN)**
• **LI, Yajuan
Shanghai, 200335 (CN)**

(74) Representative: **Tansley, Sally Elizabeth et al
Unilever N.V.
Unilever Patent Group
Bronland 14
6708 WH Wageningen (NL)**

(56) References cited:
**WO-A1-2012/076278     GB-A- 1 309 209**

## Description

### Field of the Invention

The present invention relates to oral care compositions such as tooth pastes, gums, mouthwashes and the like. In particular the present invention relates to oral care compositions comprising a tooth whitening agent. The invention also relates to the use of such compositions for whitening of teeth of an individual.

### Background of the Invention

The enamel layer of the tooth is naturally an opaque white or slightly off-white colour. However, this enamel layer can become stained or discoloured.

Many products we consume have a negative impact on our teeth and mouth. Many substances can stain or reduce the whiteness of one's teeth, in particular, certain foods, tobacco products, and fluids such as tea and coffee. These staining and discolouring substances are often able to permeate the enamel layer. This problem occurs gradually over many years, but imparts a noticeable discoloration of the enamel of one's teeth.

Consumers have always had a strong desire for white teeth and many individuals are dissatisfied with their current teeth colour. This desire for whiter teeth has given rise to a growing trend in the increased use of tooth whitening products.

A variety of products are currently used for teeth whitening. Such products often comprise peroxides, abrasives or both in order to clean and whiten teeth. These types of products are often not desired since they can cause damage to teeth and gums if overused.

There is continuing need for teeth whitening with a more efficient approach. The present inventors have now found unexpectedly that an oral care composition comprising certain tooth whitening agents provides superior instant tooth whitening benefit.

### Additional Information

US 2015/0366767 A1 (Colgate-Palmolive Company) discloses a dentifrice having a pigment system which comprises (i) a dissolvable or disintegratable film comprising one or more releasable pigments which are released during brushing, and (ii) titanium dioxide, wherein the level of titanium dioxide is 0.01-0.375% by weight of the toothpaste; together with methods of making and using the same.

WO 2012/076278 A1 (Unilever) discloses an oral care composition suitable for delivering a temporary whitening effect to the surface of teeth, the composition comprising: a continuous phase comprising water or polyhydric alcohol or a mixture thereof; a tooth surface whitening agent which is dispersed in the continuous phase, and a deposition aid for the tooth surface whitening agent; characterised in that the tooth surface whitening agent is a pearlescent pigment in an amount of at least 0.1% by weight based on the total weight of the composition, the pearlescent pigment being formed by coating one or more metal oxide layer onto particles of an inorganic substrate.

The additional information above does not describe an oral care composition comprising a tooth whitening agent which comprises (a) a first pigment and (b) a second pigment, wherein the first pigment is mica coated with metal oxides, wherein the second pigment is mica coated with metal oxides and blue colourants, and wherein the first pigment and the second pigment are present in a weight ratio (a:b) of from 2:1 to 20:1.

### Test and definitions

#### Dentifrice

"Dentifrice" for the purposes of the present invention means a paste, powder, liquid, gum or other preparation for cleaning the teeth or other surfaces in the oral cavity.

#### Tooth Paste

"Tooth paste" for the purpose of the present invention means a paste or gel dentifrice for use with a toothbrush. Especially preferred are tooth pastes suitable for cleaning teeth by brushing for about two minutes.

#### Mouth Wash

"Mouth wash" for the purpose of the present invention means liquid dentifrice for use in rinsing the mouth.

Especially preferred are mouth washes suitable for rinsing the mouth by swishing and/or gargling for about half a minute before expectorating.

## Particle Size

"Particle size" for the purpose of the present invention means D50 particle size. The D50 particle size of a particulate material is the particle size diameter at which 50 wt% of the particles are larger in diameter and 50 wt% are smaller in diameter. "Diameter", as used herein, means the largest measurable distance on a particle in the event a well-defined sphere is not generated. For the purpose of the present invention, particle sizes and distribution are measured using Malvern Mastersizer 2000 and Malvern ZetaSizer Nano series.

## pH

pH is quoted at atmospheric pressure and a temperature of 25°C. When referring to the pH of an oral care composition, this means the pH measured when 5 parts by weight of the composition is uniformly dispersed and/or dissolved in 20 parts by weight pure water at 25°C. In particular the pH may be measured by manually mixing 5 g oral care composition with 20 mL water for 30 s, then immediately testing the pH with indicator or a pH meter.

## Solubility

"Soluble" and "insoluble", as used herein, refers to the solubility of a source (e.g., like calcium salts) in water at 25°C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Slightly soluble", therefore, is defined to mean a source that dissolves in water to give a solution with a concentration of greater than 0.001 moles per litre and less than 0.1 moles per litre.

## Viscosity

Viscosity of a tooth paste is the value taken at room temperature (25°C) with a Brookfield Viscometer, Spindle No.4 and at a speed of 5 rpm. Values are quoted in centipoises (cP=mPa.s) unless otherwise specified.

## Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified. It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

## Summary of the Invention

In a first aspect, the present invention is directed to an oral care composition comprising a tooth whitening agent and a physiologically acceptable carrier; wherein the tooth whitening agent comprises:

(a) a first pigment; and
(b) a second pigment;

wherein the first pigment is mica coated with metal oxides;
wherein the second pigment is mica coated with metal oxides and blue colourants; and wherein the first pigment and the second pigment are present in a weight ratio (a:b) of from 2:1 to 20:1.

In a second aspect, the present invention is directed to a packaged oral care product comprising the oral care composition of the first aspect of this invention.

In a third aspect, the present invention is directed to a method of whitening teeth of an individual comprising the step of applying the oral care composition of any embodiment of the first aspect to at least one surface of the teeth of the individual.

All other aspects of the present invention will more readily become apparent upon considering the detailed description and examples which follow.

## Detailed Description of the Invention

The tooth whitening agent according to the invention comprises a combination of a first pigment and a second pigment. The two pigments are preferably present in an amount of at least 90% by weight of the tooth whitening agent, more preferably at least 95%, even more preferably at least 98% and most preferably the tooth whitening agent consists (or at least consists essentially) of the two pigments.

The first pigment is a pearlescent pigment which is formed by coating one or more metal oxide layers onto natural or synthetic mica flakes. The term "pearlescent", as used herein, means a pigment in the form of particles which each reflect and partially transmit the incident light. The only limitation with respect to the first pigment is that the same is suitable for use in the mouth.

Examples of suitable mica that may be used in the invention include, for example, muscovite, phlogopite, fluorophlogopite, biotite or mixtures thereof.

Illustrative yet non-limiting examples of the types of metal oxides that may be used in the invention include, for example, $TiO_2$, $Fe_2O_3$, $TiFe_2O_5$, $Fe_3O_4$, $Cr_2O_3$, $Al_2O_3$, $SiO_2$, $ZrO_2$, $ZnO$, $SnO_2$, $CoO$, $Co_3O_4$, $VO_2$, $V_2O_3$, $Sn(Sb)O_2$, titanium suboxides or mixtures thereof. $TiO_2$ is particularly preferred.

The most preferred first pigment suitable for use in the present invention comprises or is mica coated with $TiO_2$. The colour of the first pigment is preferably silver, white or mixtures thereof. Such materials are commercially available, for example, from suppliers like BASF under the trade name Timica® Soft Luster White 6500.

Typically, the first pigment has a D50 particle size of from 100 nm to 30 microns, more preferably from 1 micron to 15 microns, more preferably still from 1 micron to 10 microns, and most preferably from 2 microns to 8 microns.

The first pigment preferably comprises from 15 to 75% by weight of the metal oxide, more preferably from 25 to 65%, and most preferably from 35 to 55%, based on the total weight of the first pigment and including all ranges subsumed therein.

Typically, the oral care composition of the present invention comprises from 0.01 to 10% by weight of the first pigment, more preferably from 0.02 to 5%, and most preferably from 0.05 to 3% based on the total weight of the oral care composition and including all ranges subsumed therein.

The second pigment is also a pearlescent pigment which is formed by coating one or more metal oxide layers and other absorption colourants onto natural or synthetic mica flakes. The term "pearlescent", as used herein, means a pigment in the form of particles which each reflect and partially transmit the incident light. The only limitation with respect to the second pigment is that the same is suitable for use in the mouth.

Examples of suitable mica that may be used in the invention include, for example, muscovite, phlogopite, fluorophlogopite, biotite or mixtures thereof.

Illustrative yet non-limiting examples of the types of metal oxides that may be used in the invention include, for example, $TiO_2$, $Fe_2O_3$, $TiFe_2O_5$, $Fe_3O_4$, $Cr_2O_3$, $Al_2O_3$, $SiO_2$, $ZrO_2$, $ZnO$, $SnO_2$, $CoO$, $Co_3O_4$, $VO_2$, $V_2O_3$, $Sn(Sb)O_2$, titanium suboxides or mixtures thereof. $TiO_2$ is particularly preferred.

The other absorption colorants are blue colourants which can be precipitated on top of or simultaneously with the metal oxide layer. Examples of blue colorants that may be used in the invention include, for example, ferric ferrocyanide, cobalt blue, copper phthalocyanine or mixtures thereof. Ferric ferrocyanide is particularly preferred.

The most preferred second pigment suitable for use in the present invention comprises or is mica coated with $TiO_2$ and ferric ferrocyanide. The colour of the second pigment is preferably blue or silver-blue. Such materials are commercially available, for example, from suppliers like Merck under the trade name Colorona® Dark Blue.

Typically, the second pigment has a D50 particle size of from 1 micron to 60 microns, more preferably from 5 microns to 50 microns, more preferably still from 10 microns to 40 microns, and most preferably from 12 to 30 microns.

The second pigment preferably comprises from 10 to 80% by weight of the metal oxide, more preferably from 20 to 70%, and most preferably from 30 to 50%, based on the total weight of the second pigment and including all ranges subsumed therein.

The second pigment preferably comprises from 1 to 30% by weight of the blue colourant, preferably from 2 to 20%, and most preferably from 5 to 10%, based on total weight of the second pigment and including all ranges subsumed therein.

Typically, the oral care composition of the present invention comprises from 0.01 to 10% by weight of the second pigment, more preferably from 0.02 to 5%, and most preferably from 0.05 to 3% based on the total weight of the oral care composition and including all ranges subsumed therein.

The weight ratio of the first pigment to the second pigment in the oral care composition of the present invention ranges from 2:1 to 20:1, preferably from 2:1 to 15:1, and more preferably from 3:1 to 15:1, and most preferably from 3:1 to 10:1.

Typically, the oral care composition of the present invention comprises the first pigment and the second pigment in a total amount of from 0.01 to 10% by weight of the composition, more preferably from 0.05 to 5%, and most preferably from 0.1 to 3%, based on the total weight of the oral care composition and including all ranges subsumed therein.

Preferably, the oral care composition has a pH from 4.0 to 10.0, more preferably from 5.0 to 8.0, and most preferably from 5.5 to 7.5.

The composition of the present invention is an oral care composition and typically comprises a physiologically acceptable carrier. Water is the most common carrier for this invention. The carrier may further comprise at least surfactant, thickener, humectant or a combination thereof.

Surfactant may be used in this invention. Preferably the composition comprises at least 0.01% surfactant by weight of the composition, more preferably at least 0.1% and most preferably from 0.5 to 7%. Suitable surfactants include anionic surfactants, such as the sodium, magnesium, ammonium or ethanolamine salts of $C_8$ to $C_{18}$ alkyl sulphates (for example sodium lauryl sulphate), $C_8$ to $C_{18}$ alkyl sulphosuccinates (for example dioctyl sodium sulphosuccinate), $C_8$ to $C_{18}$ alkyl sulphoacetates (such as sodium lauryl sulphoacetate), $C_8$ to $C_{18}$ alkyl sarcosinates (such as sodium lauryl sarcosinate), $C_8$ to $C_{18}$ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate, sodium coco sulfate, cocamidopropyl betaine, sodium methyl cocoyl taurate or mixtures thereof.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, xanthan gum and/or sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another especially preferred embodiment, the thickener is xanthan gum.

Thickener typically makes up from 0.01 to about 10%, more preferably from 0.1 to 9%, and most preferably, from 0.1 to 5% by weight of the oral care composition, based on total weight of the composition and including all ranges subsumed therein.

When the oral care composition of this invention is a toothpaste or gel, the same typically has a viscosity from about 30,000 to 180,000 centipoise, and preferably, from 60,000 to 170,000 centipoise, and most preferably, from 65,000 to 165,000 centipoise.

Suitable humectants may also be used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. More preferably, the carrier humectant makes up from 25 to 80%, and most preferably, from 30 to 60% by weight of the composition, based on total weight of the composition and including all ranges subsumed therein.

The oral care composition of the present invention may contain a variety of other ingredients which are common in the art to enhance physical properties and performance. These ingredients include antimicrobial agents, anti-inflammatory agents, anti-caries agents, plaque buffers, fluoride sources, vitamins, plant extracts, desensitizing agents, anti-calculus agents, biomolecules, flavors, proteinaceous materials, preservatives, opacifying agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

The oral care composition of this invention can be used in a method of whitening the teeth of an individual comprising applying the composition to at least one surface of the teeth of the individual. The oral care composition of this invention may additionally or alternatively be for use as a medicament and/or used in the manufacture of a medicament for providing an oral care benefit as described herein, such as for whitening the teeth of an individual. Alternatively and preferably, the use is non-therapeutic.

Typically, the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The composition can be effective even when used in an individual's daily oral hygiene routine. For example, the composition may be brushed onto the teeth and/or be rinsed around the inside of the mouth of the individual. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day.

The following examples are provided to facilitate an understanding of the present invention. The examples are not provided to limit the scope of the claims.

**Examples**

Example 1

This example demonstrated the improvement of tooth whitening effect by using a combination of pigments. All ingredients are expressed by weight percent of the total formulation.

TABLE 1

| Ingredient | Samples | | | | |
|---|---|---|---|---|---|
| | 1 | 2 | 3 | 4 | 5 |
| Water | Balance | Balance | Balance | Balance | Balance |
| Disodium EDTA | 0.10 | 0.10 | 0.10 | 0.10 | 0.10 |
| Phenoxyethanol | 0.40 | 0.40 | 0.40 | 0.40 | 0.40 |
| Iodopropynyl butylcarbamate (10%) | 0.05 | 0.05 | 0.05 | 0.05 | 0.05 |
| Sodium acrylates copolymer and lecithin | 1.75 | 1.75 | 1.75 | 1.75 | 1.75 |
| Caprylic/Capric triglyceride | 6.00 | 6.00 | 6.00 | 6.00 | 6.00 |
| Dicaprylyl carbonate | 4.00 | 4.00 | 4.00 | 4.00 | 4.00 |
| Vitamin E | 0.20 | 0.20 | 0.20 | 0.20 | 0.20 |
| Colorona® Dark Blue[a] (blue mica) | 1.00 | -- | 0.10 | 0.25 | 0.50 |
| Timica® Soft Luster White 6500[b] (white mica)[b] | -- | 1.00 | 0.90 | 0.75 | 0.50 |
| a. Commercially available mica coated with titanium dioxide and ferric ferrocyanide under the trade name Colorona® Dark Blue from Merck. b. Commercially available mica coated with titanium dioxide under the trade name Timica® Soft Luster White 6500 from BASF. | | | | | |

*Methods*

Pigments were dispersed well in the test samples and could form a film with even thickness by film applicator.

The test sample was applied to a colour substrate (Leneta Company) by a cube film applicator (Ref.1103, Sheen) to form a film with a thickness of 37 $\mu$m. The colour substrate had six different colour tones from light to dark. Colour tone 3 (L* 73.12, a* 18.35, b* 27.12) was selected as the substrate for the test samples because the colour was similar to that of the native tooth enamel. DigiEye (VeriVide, England) with Nikon camera D90 in D65 light source (diffusing mode) was used to take picture of films on the colour substrate after the films were dried for 15 minutes.

A WIO index is an index which has been optimized specifically for the evaluation of whiteness in teeth (as described in Journal of Dentistry, volume 36, Supplement 1, 2008, pages 2 to 7). The WIO value of the product film on the substrate was calculated by using the CIE tristimulus values X, Y and Z with the following formula (Journal of Dentistry, 37S, 2009, e21-e26):

$$WIO = Y + 1075.012 \, (x_n - x) + 145.516 \, (y_n - y)$$

Where $(x, y)$ and $(x_n, y_n)$ are the chromaticity coordinates of the sample and the reference white respectively.

Five spots of the substrate (each with 100 x 80 pixels) were randomly selected and measured. The changes in WIO values ($\Delta$WIO) from baseline were calculated and statistically analyzed.

*Results*

The results are summarized in Table 2 (error represents standard error for duplicate measurements).

**TABLE 2**

| Change in WIO | Samples | | | | |
|---|---|---|---|---|---|
| | **1** | **2** | **3** | **4** | **5** |
| Colour tone 3 | 2.66±1.08 | 8.98±0.50 | 16.55±0.50 | 12.66±0.92 | 6.47±2.06 |

Samples 1 and 2 were comparative examples comprising only one pigment. It can be seen from the results that Samples 3 and 4 comprising a combination of two pigments showed superior tooth whitening efficacy to other samples. Sample 5, which comprised the first pigment and the second pigment in a weight ratio of 1:1, showed comparable tooth whitening efficacy to Sample 2.

**Claims**

1. An oral care composition comprising a tooth whitening agent and a physiologically acceptable carrier; wherein the tooth whitening agent comprises:

   (a) a first pigment; and
   (b) a second pigment;
   wherein the first pigment is mica coated with metal oxides;
   wherein the second pigment is mica coated with metal oxides and blue colourants; and
   wherein the first pigment and the second pigment are present in a weight ratio (a:b) of from 2:1 to 20:1.

2. The oral care composition according to claim 1, wherein mica comprises muscovite, phlogopite, fluorophlogopite, biotite or mixtures thereof.

3. The oral care composition according to claim 1 or claim 2, wherein the metal oxide comprises $TiO_2$, $Fe_2O_3$, $TiFe_2O_5$, $Fe_3O_4$, $Cr_2O_3$, $Al_2O_3$, $SiO_2$, $ZrO_2$, $ZnO$, $SnO_2$, $CoO$, $Co_3O_4$, $VO_2$, $V_2O_3$, $Sn(Sb)O_2$, titanium suboxides or mixtures thereof, preferably $TiO_2$.

4. The oral care composition according to any of the preceding claims, wherein the blue colourant comprises ferric ferrocyanide, cobalt blue, copper phthalocyanine or mixtures thereof, preferably ferric ferrocyanide.

5. The oral care composition according to any of the preceding claims, wherein the first pigment is mica coated with $TiO_2$.

6. The oral care composition according to any of the preceding claims, wherein the second pigment is mica coated with $TiO_2$ and ferric ferrocyanide.

7. The oral care composition according to any of the preceding claims, wherein the first pigment has a D50 particle size of from 100 nm to 30 microns, preferably from 1 micron to 15 microns.

8. The oral care composition according to any of the preceding claims, wherein the second pigment has a D50 particle size of from 1 micron to 60 microns, preferably 5 to 50 microns.

9. The oral care composition according to any of the preceding claims, wherein the first pigment and the second pigment are present in a weight ratio (a:b) ranging from 2:1 to 15:1, preferably from 3:1 to 15:1.

10. The oral care composition according to any of the preceding claims, wherein the first pigment comprises from 15 to 75% by weight of the metal oxide, preferably from 25 to 65%.

11. The oral care composition according to any of the preceding claims, wherein the second pigment comprises from 10 to 80% by weight of the metal oxide, preferably from 20 to 70%.

12. The oral care composition according to any of the preceding claims, wherein the second pigment comprises from 1 to 30% by weight of the blue colourant, preferably from 2 to 20%.

13. The oral care composition according to any of the preceding claims, wherein the composition comprises from 0.01 to 10% by weight of the first pigment, preferably from 0.02 to 5%.

14. The oral care composition according to any of the preceding claims, wherein the composition comprises the first pigment and the second pigment in a total amount of from 0.01 to 10% by weight of the composition, preferably from 0.05 to 5%.

15. A method of whitening teeth of an individual comprising the steps of applying the composition according to any of the preceding claims to at least one surface of the teeth of an individual.

**Patentansprüche**

1. Mundpflegezusammensetzung, umfassend ein Zahnaufhellungsmittel und einen physiologisch verträglichen Träger; wobei das Zahnaufhellungsmittel umfasst:

    (a) ein erstes Pigment; und
    (b) ein zweites Pigment;
    wobei das erste Pigment Glimmer ist, der mit Metalloxiden beschichtet ist;
    wobei das zweite Pigment Glimmer ist, der mit Metalloxiden und blauen farbgebenden Mitteln beschichtet ist; und
    wobei das erste Pigment und das zweite Pigment in einem Gewichtsverhältnis (a:b) von 2:1 bis 20:1 vorliegen.

2. Mundpflegezusammensetzung nach Anspruch 1, wobei Glimmer Muskovit, Phlogopit, Fluorphlogopit, Biotit oder Mischungen davon umfasst.

3. Mundpflegezusammensetzung nach Anspruch 1 oder Anspruch 2, wobei das Metalloxid $TiO_2$, $Fe_2O_3$, $TiFe_2O_5$, $Fe_3O_4$, $Cr_2O_3$, $Al_2O_3$, $SiO_2$, $ZrO_2$, $ZnO$, $SnO_2$, $CoO$, $Co_3O_4$, $VO_2$, $V_2O_3$, $Sn(Sb)O_2$, Titansuboxide oder Mischungen davon, vorzugsweise $TiO_2$ umfasst.

4. Mundpflegezusammensetzung nach irgendeinem der vorhergehenden Ansprüche, wobei das blaue farbgebende Mittel Eisenhexacyanidoferrat, Cobaltblau, Kupferphthalocyanin oder Mischungen davon, bevorzugt Eisenhexacyanidoferrat, umfasst.

5. Mundpflegezusammensetzung nach irgendeinem der vorhergehenden Ansprüche, wobei das erste Pigment Glimmer ist, der mit $TiO_2$ beschichtet ist.

**6.** Mundpflegezusammensetzung nach irgendeinem der vorhergehenden Ansprüche, wobei das zweite Pigment Glimmer ist, der mit $TiO_2$ und Eisenhexacyanidoferrat beschichtet ist.

**7.** Mundpflegezusammensetzung nach irgendeinem der vorhergehenden Ansprüche, wobei das erste Pigment eine D50 Teilchengröße von 100 nm bis 30 Mikron, bevorzugt von 1 Mikron bis 15 Mikron, aufweist.

**8.** Mundpflegezusammensetzung nach irgendeinem der vorhergehenden Ansprüche, wobei das zweite Pigment eine D50 Teilchengröße von 1 Mikron bis 60 Mikron, bevorzugt 5 bis 50 Mikron, aufweist.

**9.** Mundpflegezusammensetzung nach irgendeinem der vorhergehenden Ansprüche, wobei das erste Pigment und das zweite Pigment in einem Gewichtsverhältnis (a:b) im Bereich von 2:1 bis 15:1, bevorzugt von 3:1 bis 15:1, vorliegen.

**10.** Mundpflegezusammensetzung nach irgendeinem der vorhergehenden Ansprüche, wobei das erste Pigment 15 bis 75 Gewichts-% des Metalloxids, bevorzugt 25 bis 65%, umfasst.

**11.** Mundpflegezusammensetzung nach irgendeinem der vorhergehenden Ansprüche, wobei das zweite Pigment 10 bis 80 Gewichts-% des Metalloxids, bevorzugt 20 bis 70%, umfasst.

**12.** Mundpflegezusammensetzung nach irgendeinem der vorhergehenden Ansprüche, wobei das zweite Pigment 1 bis 30 Gewichts-% des blauen farbgebenden Mittels, bevorzugt 2 bis 20%, umfasst.

**13.** Mundpflegezusammensetzung nach irgendeinem der vorhergehenden Ansprüche, wobei die Zusammensetzung 0,01 bis 10 Gewichts-% des ersten Pigments, bevorzugt 0,02 bis 5%, umfasst.

**14.** Mundpflegezusammensetzung nach irgendeinem der vorhergehenden Ansprüche, wobei die Zusammensetzung das erste Pigment und das zweite Pigment in einer Gesamtmenge von 0,01 bis 10 Gewichts-% der Zusammensetzung, bevorzugt 0,05 bis 5%, umfasst.

**15.** Verfahren zum Aufhellen von Zähnen eines Individuums, umfassend die Schritte des Aufbringens der Zusammensetzung nach irgendeinem der vorhergehenden Ansprüche auf mindestens eine Oberfläche der Zähne eines Individuums.

## Revendications

**1.** Composition de soins bucco-dentaires comprenant un agent de blanchiment des dents et un vecteur physiologiquement acceptable ; dans laquelle l'agent de blanchiment des dents comprend :

    (a) un premier pigment ; et
    (b) un second pigment ;
    dans laquelle le premier pigment est du mica revêtu avec des oxydes métalliques ;
    dans laquelle le second pigment est du mica revêtu avec des oxydes métalliques et des colorants bleus ; et
    dans laquelle le premier pigment et le second pigment sont présents dans un rapport pondéral (a:b) de 2:1 à 20:1.

**2.** Composition de soins bucco-dentaires selon la revendication 1, dans laquelle le mica comprend de la muscovite, de la phlogopite, de la fluorophlogopite, de la biotite ou des mélanges de celles-ci.

**3.** Composition de soins bucco-dentaires selon la revendication 1 ou la revendication 2, dans laquelle l'oxyde métallique comprend $TiO_2$, $Fe_2O_3$, $TiFe_2O_5$, $Fe_3O_4$, $Cr_2O_3$, $Al_2O_3$, $SiO_2$, $ZrO_2$, $ZnO$, $SnO_2$, $CoO$, $Co_3O_4$, $VO_2$, $V_2O_3$, $Sn(Sb)O_2$, des sous-oxydes de titane ou des mélanges de ceux-ci, de préférence $TiO_2$.

**4.** Composition de soins bucco-dentaires selon l'une quelconque des revendications précédentes, dans laquelle le colorant bleu comprend du ferrocyanure ferrique, du bleu de cobalt, de la phtalocyanine de cuivre ou des mélanges de ceux-ci, de préférence du ferrocyanure ferrique.

**5.** Composition de soins bucco-dentaires selon l'une quelconque des revendications précédentes, dans laquelle le premier pigment est du mica revêtu avec $TiO_2$.

6. Composition de soins bucco-dentaires selon l'une quelconque des revendications précédentes, dans laquelle le second pigment est du mica revêtu avec $TiO_2$ et du ferrocyanure ferrique.

7. Composition de soins bucco-dentaires selon l'une quelconque des revendications précédentes, dans laquelle le premier pigment a une taille de particules D50 de 100 nm à 30 microns, de préférence de 1 micron à 15 microns.

8. Composition de soins bucco-dentaires selon l'une quelconque des revendications précédentes, dans laquelle le second pigment a une taille de particules D50 de 1 micron à 60 microns, de préférence de 5 à 50 microns.

9. Composition de soins bucco-dentaires selon l'une quelconque des revendications précédentes, dans laquelle le premier pigment et le second pigment sont présents dans un rapport pondéral (a:b) allant de 2:1 à 15:1, de préférence de 3:1 à 15:1.

10. Composition de soins bucco-dentaires selon l'une quelconque des revendications précédentes, dans laquelle le premier pigment comprend de 15 à 75 % en poids de l'oxyde métallique, de préférence de 25 à 65 %.

11. Composition de soins bucco-dentaires selon l'une quelconque des revendications précédentes, dans laquelle le second pigment comprend de 10 à 80 % en poids de l'oxyde métallique, de préférence de 20 à 70 %.

12. Composition de soins bucco-dentaires selon l'une quelconque des revendications précédentes, dans laquelle le second pigment comprend de 1 à 30 % en poids du colorant bleu, de préférence de 2 à 20 %.

13. Composition de soins bucco-dentaires selon l'une quelconque des revendications précédentes, dans laquelle la composition comprend de 0,01 à 10 % en poids du premier pigment, de préférence de 0,02 à 5 %.

14. Composition de soins bucco-dentaires selon l'une quelconque des revendications précédentes, dans laquelle la composition comprend le premier pigment et le second pigment en une quantité totale de 0,01 à 10 % en poids de la composition, de préférence de 0,05 à 5 %.

15. Procédé de blanchiment des dents d'un individu comprenant les étapes d'application de la composition selon l'une quelconque des revendications précédentes sur au moins une surface des dents d'un individu.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150366767 A1

- WO 2012076278 A1

**Non-patent literature cited in the description**

- *CHEMICAL ABSTRACTS,* 9063-87-0
- *CHEMICAL ABSTRACTS,* 9004-32-4

- *Journal of Dentistry,* 2008, vol. 36, 2-7
- *Journal of Dentistry,* 2009, vol. 37S, e21-e26